# FASCICULE DE BREVET EUROPEEN

(11) **EP 0 561 441 B1**
(45) Date de publication et mention de la délivrance du brevet: **06.11.1996**
(21) Numéro de dépôt: 93200566.3
(22) Date de dépôt: 01.03.1993
(51) Int. Cl.: C07C 29/76, C07C 29/86, C07C 31/36, C07D 301/02, C07D 301/24, C07D 301/26, C07D 301/32

(54) **Procédé de production d'épichlorhydrine**
Verfahren zur Herstellung von Epichlorhydrin
Process for the manufacture of epichlorohydrin

(30) Priorité: 17.03.1992 BE 9200259
(43) Date de publication de la demande: 22.09.1993
(73) Titulaire: SOLVAY (Société Anonyme), B-1050 Bruxelles (BE)
(72) Inventeur: Grunchard, Frans, B-3090 Overijse (BE)
(74) Mandataire: Marckx, Frieda

(56) Documents cités:
- EP-A- 0 359 331
- EP-A- 0 537 846
- CHEMICAL ABSTRACTS, vol. 107, no. 8, 24 Août 1987, Columbus, Ohio, US; abstract no. 61007, V. SAULI, SCHEJBAL I. 'Epichlorohydrin manufacture' page 109 ;
- CHEMICAL ABSTRACTS, vol. 69, no. 22, 25 Novembre 1968, Columbus, Ohio, US; abstract no. 90301, URBANCOVA, L. 'Liquid-liquid equilibrium in systems 1,3- dichloro-2-propanol-1,2,3-t richloropropane-water.' page 8461 ;
- DATABASE WPI Week 7541, 1975 Derwent Publications Ltd., London, GB; AN 75-68268W & JP-B-50 028 408 (OSAKA) 16 Septembre 1975
- DATABASE WPI Week 8137, 1981 Derwent Publications Ltd., London, GB; AN 81- 67350D & SU-A-789 479 (SHAROV V G) 24 Décembre 1980

## Description

La présente invention se rapporte à un procédé de production d'épichlorhydrine par déshydrochloration de dichloropropanols, eux-mêmes obtenus par hypochloration du chlorure d'allyle et, plus particulièrement, à un procédé amélioré dont les effluents aqueux sont appauvris en impuretés organiques chlorées.

Il est bien connu de préparer l'épichlorhydrine par déshydrochloration, à l'aide d'un composé basique, d'une solution aqueuse de dichloropropanols, laquelle est obtenue en faisant réagir, dans une zone réactionnelle appropriée, du chlorure d'allyle, de l'eau et du chlore.

Au cours de la réaction d'hypochloration du chlorure d'allyle en dichloropropanols se forment généralement de nombreux sous-produits non désirés. Il est possible d'améliorer la sélectivité de la réaction d'hypochloration en effectuant la réaction en milieu aqueux très dilué. Cette pratique communément utilisée, provoque cependant, à l'étape ultérieure de déshydrochloration des dichloropropanols en épichlorhydrine, la formation de volumes importants d'effluents aqueux contenant des impuretés organiques à l'état fortement dilué, dont l'épuration requiert des traitements coûteux. Ces impuretés organiques sont constituées entre autres de produits organiques chlorés lourds, difficiles à éliminer et ayant comme précurseurs les sous-produits de la réaction d'hypochloration. Une diminution de la quantité des impuretés organiques chlorées présentes dans les effluents aqueux permettrait de réaliser d'importantes économies.

La demande de brevet EP-A-0359331 de Shell divulgue un procédé pour réduire la teneur en chloroalcanes et éthers chloroaliphatiques d'une solution aqueuse de dichloropropanols, par mise en contact de cette solution avec du tétrachlorométhane. Après séparation de la phase aqueuse, le solvant chargé en impuretés est lavé à l'eau pour en retirer les dichloropropanols également extraits puis régénéré par distillation et recyclé à l'étape d'extraction. Ce procédé connu présente l'inconvénient de comporter obligatoirement une étape de regénération du solvant mis en oeuvre, entraînant un accroissement de la consommation énergétique. En outre, une certaine fraction du solvant est inévitablement perdue à différentes étapes du procédé, impliquant dès lors l'addition régulière de solvant frais afin de conserver un rapport volumique suffisant entre le solvant d'extraction et la solution aqueuse brute de dichloropropanols à traiter. Ce procédé implique en outre la mise en oeuvre d'un produit extérieur au procédé et, il nécessite des étapes supplémentaires de reconversion de l'épichlorhydrine en dichloropropanols. Dans le brevet US-A-2,873,298 de Shell, la majeure partie du produit aqueux d'hypochloration du chlorure d'allyle en dichloropropanols est envoyée avec un léger excès d'hydroxyde de sodium dans une colonne d'entraînement à la vapeur. La solution brute d'épichlorhydrine obtenue par déshydrochloration, qui contient du trichloropropane et des impuretés organiques non identifiées, est diluée avec le solde de la solution aqueuse de dichloropropanols.

Cette solution diluée est soumise à une décantation. La phase organique qui contient la majeure partie du trichloropropane et des impuretés organiques non identifiées, ainsi que des quantités appréciables d'épichlorhydrine et de dichloropropanols est soumise à une étape de conversion de l'épichlorhydrine en monochlorhydrine ou à une étape de distillation de l'épichlorhydrine. Ces étapes sont suivies de la récupération des mono- et/ou dichloropropanols par extraction à l'eau et les phases organiques résiduelles contenant alors principalement le trichloropropane et les impuretés organiques non identifiées sont écartées. Ce procédé n'améliore pas la qualité des effluents aqueux de l'étape de déshydrochloration des dichloropropanols.

Le certificat d'auteur SU-A-789 479 (SHAROV V.G.) décrit un procédé de préparation de dichloropropanols par hypochloration du chlorure d'allyle en présence d'alcool allylique et de 1,2,3-trichloropropane, qui ont pour fonction d'empêcher la formation de sous-produits.

La demande de brevet EP-A-0 537 846 (SHELL), publiée après la date de dépôt de la présente demande de brevet et citée comme faisant partie de l'état de la technique en vertu de l'article 54(3) et (4) CBE, concerne un procédé de production de dichloropropanols comprenant une étape d'hypochloration du chlorure d'allyle pour former une solution aqueuse brute de dichloropropanols qui contient en outre de faibles quantités d'impuretés organiques chlorées, et une étape d'extraction de cette solution aqueuse brute de dichloropropanols avec du 1,2,3-trichloropropane pour obtenir une phase organique formée par le 1,2,3-trichloropropane enrichi en impuretés chloroaliphatiques et une phase aqueuse contenant la majeure partie des dichloropropanols.

L'invention vise à remédier aux inconvénients des procédés connus de préparation d'épichlorhydrine en fournissant un procédé de préparation d'épichlorhydrine au départ de chlorure d'allyle, via les dichloropropanols, comportant une étape d'épuration de la solution aqueuse brute de dichloropropanols par extraction liquide-liquide au moyen d'un solvant d'extraction nettement plus efficace que le tétrachlorométhane. Les rendements sont très bons, le procédé est simple à réaliser et il permet de réduire les consommations d'énergie. Les effluents aqueux directs du procédé contiennent très peu d'impuretés organiques chlorées, ce qui permet d'éviter certaines étapes ultérieures trop complexes ou trop consommatrices d'énergie pour les épurer avant de les rejeter. La pureté de l'épichlorhydrine obtenue est très bien adaptée aux usages ultérieurs du produit sans que des étapes supplémentaires coûteuses et consommatrices d'énergie soient nécessaires. En outre, les impuretés organiques chlorées sont concentrées et peuvent dès lors être traitées de façon économique, soit en vue de leur valorisation ultérieure, soit en vue de leur élimination.

En conséquence, l'invention concerne un procédé de production d'épichlorhydrine au départ de chlorure d'allyle, comportant une étape d'hypochloration du chlorure d'allyle pour former une solution aqueuse brute de dichloropropanols, une étape d'épuration de la solution aqueuse brute de dichloropropanols en impuretés organiques chlorées pour former une solution aqueuse épurée de dichloropropanols, et une étape d'hydrolyse des dichloropropanols, comment décrit dans la reivindication 1

Les impuretés organiques chlorées présentes dans la solution aqueuse brute de dichloropropanols obtenue par hypochloration du chlorure d'allyle en dichloropropanols comprennent essentiellement, d'une part, des alcanes chlorés, dont principalement le 1,2,3-trichloropropane et, d'autre part, d'autres impuretés appelées ci-après impuretés chloroaliphatiques qui comprennent principalement des éthers chloroaliphatiques tels que notamment des polychlorodipropyléthers, des polychlorohexylpropyléthers, et des polychlorononylpropyléthers et des alcools chloroaliphatiques tels que notamment des polychlorohexanols ou -diols et des polychlorononanols.

Par solvant organique riche en 1,2,3-trichloropropane, on entend, aux fins de la présente invention, un solvant comprenant au moins environ 30 % en poids de 1,2,3-trichloropropane. De préférence, ce solvant organique recyclé comprend au moins environ 50 % en poids de 1,2,3-trichloropropane. Bien que le procédé selon l'invention peut utiliser un solvant organique recyclé constitué uniquement de 1,2,3-trichloropropane, en pratique, on utilise comme solvant organique d'extraction recyclé, un mélange de produits pouvant comprendre jusqu'à environ 95 % de 1,2,3-trichloropropane, le solde étant constitué par divers composés classiquement mis en oeuvre ou formés lors de la synthèse d'épichlorhydrine au départ de chlorure d'allyle. La nature des composés autres que le 1,2,3-trichloropropane présents dans le solvant organique recyclé et leur teneur dépendent du mode de mise en oeuvre du procédé selon l'invention. En règle générale, ces composés sont principalement des dichloropropanols. Le solvant organique recyclé peut comprendre de 0,5 à 60 % en poids de dichloropropanols.

Le solvant organique recyclé peut aussi éventuellement comprendre de légères quantités d'épichlorhydrine, par exemple, de 0,1 à 5 % en poids et de faibles quantités d'impuretés chloroaliphatiques, par exemple de 0,1 à 5 % en poids.

Le procédé selon l'invention apparaît particulièrement avantageux. En effet, la majorité des impuretés chloroaliphatiques présentes dans la solution aqueuse brute de dichloropropanols sont nettement plus solubles dans le 1,2,3-trichloropropane que dans les solvants d'extraction utilisés dans les procédés connus, tels que le tétrachlorométhane. Le 1,2,3-trichloropropane s'avère dès lors être un solvant particulièrement efficace pour extraire les impuretés chloroaliphatiques telles que les éthers et alcools chloroaliphatiques présents dans la solution aqueuse brute de dichloropropanols. La mise en contact de la solution aqueuse brute de dichloropropanols avec un solvant organique recyclé, riche en 1,2,3-trichloropropane, conduit, après extraction, à une solution aqueuse épurée de dichloropropanols, fortement appauvrie en impuretés chloroaliphatiques, particulièrement en éthers et alcools chloroaliphatiques.

Par rapport au procédé connu mettant en oeuvre un solvant d'extraction tel que le tétrachlorométhane, la solution aqueuse épurée de dichloropropanols obtenue dans le procédé selon l'invention renferme des quantités plus importantes de 1,2,3-trichloropropane. De manière surprenante, la présence de ces quantités de 1,2,3-trichloropropane n'affecte pratiquement pas, à l'étape ultérieure de déshydrochloration des dichloropropanols, la teneur en impuretés organiques chlorées des effluents aqueux, lesquels sont donc globalement fortement épurés en impuretés organiques chlorées générées à l'étape d'hypochloration du chlorure d'allyle.

Du 1,2,3-trichloropropane étant habituellement produit en quantités non négligeables lors de l'hypochloration du chlorure d'allyle en dichloropropanols, les procédés existants disposent généralement d'un dispositif destiné à récupérer et à purifier le 1,2,3-trichloropropane en vue de sa valorisation ultérieure. Le plus souvent, la purification du 1,2,3-trichloropropane est réalisée par distillation. Le recyclage d'au moins une partie d'un flux renfermant du 1,2,3-trichloropropane issu d'une telle distillation en vue de l'utiliser comme solvant d'extraction des impuretés organiques chlorées présentes dans la solution aqueuse brute de dichloropropanols ne nécessite dès lors aucun investissement supplémentaire et pratiquement aucune augmentation de la consommation énergétique. Le procédé selon l'invention présente en outre l'avantage de réaliser en une seule étape, consistant en une distillation fractionnée, la purification du 1,2,3-trichloropropane sous-produit, la régénération du solvant d'extraction à recycler et la concentration, en vue de leur destruction, des impuretés chloroaliphatiques extraites de la solution aqueuse brute de dichloropropanols.

Dans le mode de mise en oeuvre préféré du procédé selon l'invention, l'épichlorhydrine est préparée selon un procédé comprenant les étapes suivantes :
a) hypochloration du chlorure d'allyle pour former une solution aqueuse brute de dichloropropanols qui contient en outre de faibles quantités d'impuretés organiques chlorées, notamment du 1,2,3-trichloropropane et des impuretés chloroaliphatiques.
b) extraction de cette solution aqueuse brute de dichloropropanols avec un solvant organique d'extraction recyclé, riche en 1,2,3-trichloropropane, en quantité suffisante pour obtenir une phase organique qui comprend principalement du 1,2,3-trichloropropane enrichi en impuretés chloroaliphatiques et une phase aqueuse contenant la majeure partie des dichloropropanols, fortement appauvrie en impuretés chloroaliphatiques.
c) déshydrochloration des dichloropropanols en épichlorhydrine par introduction d'un composé basique dans la phase aqueuse obtenue à l'étape b), entraînement à la vapeur de l'épichlorhydrine formée pour obtenir de l'épichlorhydrine brute et rejet des effluents aqueux.
d) séparation, par distillation fractionnée, de l'épichlorhydrine brute formée en c) en une fraction légère, en une fraction intermédiaire constituée d'épichlorhydrine purifiée et en une fraction lourde résiduelle et recyclage de la fraction lourde résiduelle de cette distillation à l'étape b) d'extraction où elle constitue au moins partiellement le solvant organique d'extraction recyclé riche en 1,2,3-trichloropropane.
e) lavage avec de l'eau, de la phase organique obtenue à l'étape b), pour obtenir, après séparation des phases, une eau de lavage contenant la majeure fraction des dichloropropanols présents dans ladite phase organique et un extrait organique lavé, contenant nettement moins de dichloropropanols qu'à l'alimentation, l'eau de lavage chargée en dichloropropanols étant recyclée à au moins une des étapes a) d'hypochloration, b) d'extraction ou c) de déshydrochloration.
f) séparation, par distillation fractionnée, dudit extrait organique lavé de l'étape e), en une coupe de tête envoyée à l'étape d) de séparation par distillation de l'épichlorhydrine brute, en une coupe moyenne constituée de 1,2,3-trichloropropane purifié et en une coupe de queue, constituée essentiellement par les impuretés chloroaliphatiques.

Ce mode de mise en oeuvre préféré du procédé permet d'évacuer du procédé les produits plus légers que l'épichlorhydrine éventuellement présents dans l'extrait organique lavé, sans devoir recourir à une purge supplémentaire. En outre, le 1,2,3-trichloropropane contenu dans la coupe de tête obtenue à l'étape f) par séparation par distillation de l'extrait organique lavé et envoyée à l'étape d) de séparation par distillation de l'épichlorhydrine brute constitue, avec les dichloropropanols non convertis et avec le 1,2,3-trichloropropane dissous dans la phase aqueuse à l'étape b) d'extraction, la quasi totalité de la fraction lourde résiduelle de la séparation par distillation de l'épichlorhydrine brute effectuée à l'étape d), laquelle fraction lourde résiduelle est recyclée à l'étape b) d'extraction, où elle constitue, dans ce mode préféré de mise en oeuvre du procédé selon l'invention, le solvant organique d'extraction recyclé, riche en 1,2,3-trichloropropane, mis en contact avec la solution aqueuse brute de dichloropropanols. Un tel agencement permet une récupération optimale de tous les produits et réactifs intervenant dans le procédé de préparation de l'épichlorhydrine. En outre, par rapport aux procédés connus cités ci-avant, ce mode de mise en oeuvre préféré du procédé selon l'invention présente l'avantage supplémentaîre de ne pas comporter d'étape de reconversion de l'épichlorhydrine en dichloropropanols.

Dans une variante de ce mode de mise en oeuvre préféré du procédé selon l'invention, une partie du 1,2,3-trichloropropane purifié, correspondant à la coupe moyenne obtenue à l'étape f) de séparation par distillation de l'extrait organique lavé, peut être ajoutée à la fraction lourde résiduelle obtenue à l'étape d) de séparation par distillation de l'épichlorhydrine brute, le mélange obtenu étant recyclé à l'étape b) d'extraction, en tant que solvant organique d'extraction recyclé.

De manière particulièrement préférée, la séparation par distillation de l'extrait organique lavé de l'étape e), effectuée à l'étape f), est menée de manière telle que la coupe de tête envoyée à l'étape d) de séparation par distillation de l'épichlorhydrine contienne de 50 à 98 % de 1,2,3-trichloropropane, le solde étant constitué par des composés plus légers que le 1,2,3-trichloropropane. Les composés plus légers que le 1,2,3-trichloropropane, c'est-à-dire ceux dont le point d'ébullition à pression normale est inférieur à 156 °C, comprennent principalement certains produits tels que des impuretés présentes dans le chlorure d'allyle et le chlorure d'allyle qui n'aurait pas réagi lors de l'hypochloration.

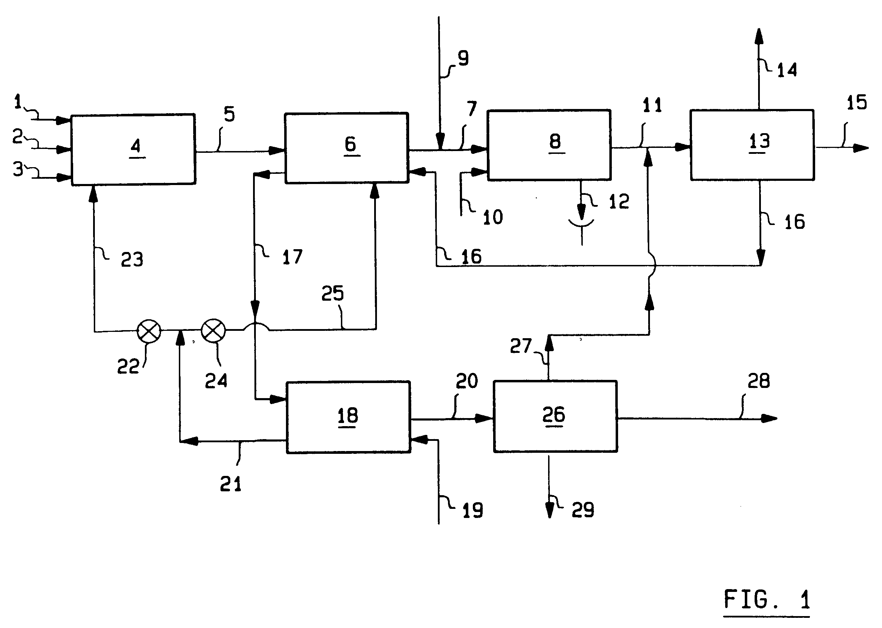

Ce mode de mise en oeuvre particulièrement préféré du procédé selon l'invention est décrit ci-après de manière détaillée en faisant référence à la figure 1, représentant schématiquement l'agencement préféré des différentes étapes.

Le chlorure d'allyle, le chlore et l'eau introduits respectivement par les conduits 1, 2 et 3 réagissent dans la zone réactionnelle 4, pour former, par hypochloration du chlorure d'allyle, des dichloropropanols ainsi que de petites quantités de nombreuses impuretés organiques chlorées. La zone réactionnelle 4 est constituée d'un ou de plusieurs réacteurs consécutifs ou disposés en parallèle, tels que des réacteurs agités, des réacteurs à recirculation, des réacteurs venturi ou tout autre type de réacteur permettant une dispersion rapide et efficace des réactifs. La réaction d'hypochloration se déroule généralement à une température de 20 à 70 °C.

Par le conduit 5, la solution aqueuse brute de dichloropropanols, contenant généralement de 2 à 12 % en poids de dichloropropanols et les impuretés organiques chlorées, est amenée à une zone d'extraction 6 où elle est mise en contact intime avec un solvant organique d'extraction recyclé, riche en 1,2,3-trichloropropane, introduit par le conduit 16. La mise en contact de la solution aqueuse brute de dichloropropanols et du solvant d'extraction est réalisée en une ou plusieurs étapes, au moyen de n'importe quel dispositif conventionnel d'extraction liquide-liquide, par exemple par mise en contact intime au moyen d'un réacteur agité, d'un extracteur à disques rotatifs, d'un extracteur à centrifugation ou d'une colonne à plateaux perforés, fonctionnant soit à contre-courant, soit à co-courant. L'extraction peut être menée en continu ou en discontinu. De préférence, elle est menée en continu. Dans ce cas, le rapport entre le débit du solvant d'extraction riche en 1,2,3-trichloropropane, introduit en 16 et le débit de la solution aqueuse brute de dichloropropanols, introduite par le conduit 5, nécessaire pour effectuer une extraction satisfaisante des impuretés chloroaliphatiques, et plus particulièrement des éthers et alcools chloroaliphatiques, est bien sûr fonction de différents paramètres, notamment du dispositif d'extraction liquide-liquide mis en oeuvre et de la teneur en 1,2,3-trichloropropane dans le solvant d'extraction. D'une manière générale, on peut toutefois affirmer que de bons résultats peuvent être obtenus lorsque ce rapport est supérieur à environ 0,001. De manière préférée, ce rapport est supérieur à environ 0,002. De manière particulièrement préférée, il est supérieur à environ 0,005. Généralement, ce rapport est inférieur à environ 0,1. De manière préférée, il est inférieur à environ 0,05. De manière particulièrement préférée, il est inférieur à environ 0,03.

Selon le dispositif d'extraction mis en oeuvre, il est éventuellement nécessaire de séparer le mélange contenant une phase aqueuse et une phase organique en ces deux phases respectives. Cette séparation peut être réalisée simplement par décantation, mais on peut aussi mettre en oeuvre tout autre dispositif classique de séparation de phases, tel qu'une centrifugation ou une séparation par hydrocyclone. La phase organique comprend principalement du 1,2,3-trichloropropane enrichi en impuretés chloroaliphatiques, mais contient aussi une quantité non négligeable de dichloropropanols. Sa composition correspond le plus souvent à la composition d'équilibre, déterminée par les coefficients de partage des différents composés entre l'eau et le 1,2,3-trichloropropane. La phase aqueuse contient la majeure partie des dichloropropanols. Elle est généralement saturée en 1,2,3-trichloropropane. Elle constitue, dans le procédé selon l'invention, la solution aqueuse épurée de dichloropropanols. La phase aqueuse obtenue dans la zone d'extraction 6 est particulièrement bien épurée en impuretés chloroaliphatiques. Elle présente une teneur résiduelle en ces impuretés extrêmement faible, impossible à atteindre uniquement par les procédés connus cités ci-avant.

La phase aqueuse, épurée en impuretés chloraliphatiques, quitte la zone d'extraction 6 par le conduit 7 et est ensuite traitée par une solution aqueuse alcaline introduite par le conduit 9, neutralisant l'acide chlorhydrique formé lors de l'hypochloration et provoquant, par déshydrochloration, la conversion des dichloropropanols en épichlorhydrine. Comme solution aqueuse alcaline, on peut utiliser, notamment, une solution d'hydroxyde de sodium, d'hydroxyde de calcium ou encore de carbonate de sodium.

La solution obtenue est alors immédiatement transférée dans une colonne d'entraînement à la vapeur 8, alimentée à sa base par un flux de vapeur 10. L'épichlorhydrine, ainsi que le 1,2,3-trichloropropane solubilisé dans la phase aqueuse à l'étape d'extraction 6, les impuretés chloroaliphatiques résiduelles, non extraites de la phase aqueuse dans la zone d'extraction 6 et certains composés relativement volatils, générés notamment lors de la déshydrochloration des dichloropropanols, sont entraînés par la vapeur en tête de la colonne d'entraînement à la vapeur 8. Après condensation des produits entraînés, ceux-ci décantent en deux phases (non représenté à la figure 1) : une fraction aqueuse moins dense, renfermant une partie des dichloropropanols non transformés et un peu d'épichlorhydrine, laquelle fraction aqueuse est renvoyée comme reflux à la colonne d'entraînement à la vapeur et une fraction organique constituant l'épichlorhydrine brute envoyée par le conduit 11 vers la zone de distillation de l'épichlorhydrine 13. Cette épichlorhydrine brute contient principalement, outre de l'épichlorhydrine, un peu d'eau, des dichloropropanols non transformés, le 1,2,3-trichloropropane dissous dans la phase aqueuse dans la zone d'extraction 6 et les impuretés chloroaliphatiques résiduelles, non extraites de la phase aqueuse dans la zone d'extraction 6.

En pied de la colonne 8, via le conduit 12, sont évacués les effluents aqueux, contenant notamment les sels minéraux générés par l'hydrolyse. Dans ces effluents, la charge en impuretés chloroaliphatiques générées à l'étape d'hypochloration du chlorure d'allyle est particulièrement faible, ce qui rend leur épuration plus facile.

La zone de distillation 13, constituée d'une ou, de préférence, de plusieurs colonnes de distillation permet d'éliminer de l'épichlorhydrine brute acheminée par le conduit 11, d'une part, une fraction légère comprenant l'eau et les composés relativement volatils, lesquels sont évacués par le conduit 14 vers une unité de traitement où ils sont généralement détruits, par exemple par combustion et, d'autre part, une fraction lourde résiduelle constituée principalement de 1,2,3-trichloropropane, de dichloropropanols non convertis et des impuretés chloroaliphatiques non extraites de la phase aqueuse dans la zone d'extraction 6. Recyclée à la zone 6 d'extraction via le conduit 16, cette fraction lourde constitue, dans ce mode préféré de mise en oeuvre du procédé selon l'invention, le solvant organique d'extraction recyclé, riche en 1,2,3-trichloropropane. Le recyclage de cette fraction lourde permet, en outre, de récupérer les dichloropropanols éventuellement non hydrolysés lors de l'étape de déshydrochloration et entraînés dans l'épichlorhydrine brute. En règle générale, cette fraction lourde peut comprendre, outre du 1,2,3-trichloropropane, de 0,5 à 60 % en poids de dichloropropanols et de 0,1 à 5 % d'impuretés chloroaliphatiques. De préférence, elle comprend de 1 à 40 % de dichloropropanols et de 0,5 à 3 % d'impuretés chloroaliphatiques.

L'épichlorhydrine purifiée, quant à elle, quitte le procédé par le conduit 15.

La phase organique obtenue dans la zone d'extraction 6 contient principalement du 1,2,3-trichloropropane, d'une part provenant du solvant organique d'extraction recyclé introduit à l'étape d'extraction 6 par le conduit 16 et d'autre part, formé lors de l'hypochloration du chlorure d'allyle dans la zone réactionelle 4 et les impuretés chloroaliphatiques, dont les éthers et alcools chloroaliphatiques, également formées lors de l'hypochloration du chlorure d'allyle. La phase organique contient cependant également des quantités non négligeables de dichloropropanols extraits de la solution aqueuse brute de dichloropropanols par le solvant organique d'extraction lors de l'étape d'extraction 6, ainsi que de petites quantités de produits tels que les impuretés du chlorure d'allyle et, éventuellement, le chlorure d'allyle non converti dans la zone réactionnelle 4.

Pour récupérer les dichloropropanols présents dans la phase organique obtenue dans la zone d'extraction 6, cette phase organique est transférée via le conduit 17 dans une zone de lavage 18 où elle est lavée à l'eau. Dans cette zone 18, le lavage est réalisé en une ou plusieurs étapes dans tout dispositif classique d'extraction liquide-liquide, par mise en contact intime de la phase organique amenée par le conduit 17 avec de l'eau, laquelle est introduite dans la zone 18 via le conduit 19. La quantité d'eau nécessaire pour effectuer ce lavage dépend de l'efficacité du dispositif utilisé. Ce lavage peut être mené en continu ou en discontinu. De préférence, il est réalisé en continu. Dans ce cas, le rapport volumique entre le flux d'eau de lavage et le flux d'extrait organique est généralement compris entre 5:1 et 20:1. Après lavage et séparation des phases, l'eau de lavage est généralement saturée en 1,2,3-trichloropropane et elle peut renfermer de 1 à 10 % en poids de dichloropropanols. Elle est envoyée, via le conduit 21, la vanne 22 et le conduit 23 à la zone de réaction 4, où elle peut remplacer une partie de l'eau introduite par le conduit 3. Si la teneur en dichloropropanols de cette eau de lavage est suffisamment élevée, elle est, de préférence, envoyée directement à la zone d'extraction 6 via le conduit 21, la vanne 24 et le conduit 25.

A la sortie de la zone 18, l'extrait organique lavé ne contient pratiquement plus de dichloropropanols. Il comprend principalement du 1,2,3-trichloropropane et généralement de 3 à 25 % d'impuretés chloroaliphatiques. Cet extrait organique lavé est transféré par le conduit 20 dans la zone de distillation 26. Dans cette zone, comprenant une ou plusieurs étapes de distillation fractionnée, l'extrait organique lavé est séparé en une coupe de tête contenant les composés plus légers que le 1,2,3-trichloropropane et du 1,2,3-trichloropropane, laquelle coupe de tête est acheminée, via les conduits 27 et 11, à la zone de distillation de l'épichlorhydrine 13, en une coupe moyenne constituée par du 1,2,3-trichloropropane de pureté de l'ordre de 99 %, laquelle coupe moyenne quitte le procédé par le conduit 28, et en une coupe de queue constituée essentiellement par les impuretés chloroaliphatiques, laquelle coupe de queue est évacuée par le conduit 29. Obtenues de cette manière sous forme concentrée, les impuretés chloroaliphatiques peuvent être éliminées de manière économique par différentes techniques connues, par exemple par combustion.

Dans le mode de mise en oeuvre particulièrement préféré du procédé selon l'invention illustré à la figure 1, la distillation réalisée en 26 est menée de manière telle que la coupe de tête récupérée par le conduit 27, contient au moins environ 50 % de 1,2,3-trichloropropane. De manière tout particulièrement préférée, sa teneur en 1,2,3-trichloropropane est supérieure à environ 70 %. De très bons résultats ont été obtenus avec une teneur en 1,2,3-trichloropropane dans cette coupe de tête de l'ordre de 80 à 98 %. Outre du 1,2,3-trichloropropane, cette coupe de tête comprend principalement les composés plus légers que le 1,2,3-trichloropropane, principalement certains produits tels que les impuretés présentes dans le chlorure d'allyle et le chlorure d'allyle qui n'aurait pas réagi lors de l'hypochloration.

L'invention concerne aussi un procédé d'extraction des impuretés chloroaliphatiques d'une solution aqueuse brute de dichloropropanols obtenue par hypochloration du chlorure d'allyle caractérisé en ce que l'extraction est réalisée au moyen d'un solvant organique d'extraction recyclé, riche en 1,2,3-trichloropropane.

L'invention concerne encore la solution aqueuse de dichloropropanols fortement épurée en impuretés chloroaliphatiques, obtenue par le procédé de l'invention.

L'invention concerne enfin l'utilisation d'un solvant d'extraction recyclé, riche en 1,2,3-trichloropropane comme agent d'extraction d'impuretés chloroaliphatiques contenues dans une solution brute de dichloropropanols.

L'exemple suivant illustre l'efficacité de l'épuration de la solution aqueuse brute de dichloropropanols selon le procédé de l'invention par comparaison avec un procédé connu.

### Exemple

Une solution aqueuse brute de dichloropropanols, obtenue par hypochloration de chlorure d'allyle, est tout d'abord décantée pour en éliminer la fraction des impuretés organiques chlorées facilement séparable.

Le tableau reprend la concentration en différentes impuretés chloroaliphatiques présentes dans la phase aqueuse après décantation, identifiées par leur formule brute, les différents isomères étant distingués par leur temps de rétention en chromatographie en phase gazeuse.

Un litre de cette phase aqueuse décantée est extrait à 50 °C par 25 ml de 1,2,3-trichloropropane (selon l'invention), et un autre litre par 25 ml de tétrachlorométhane à titre de comparaison.

Le tableau donne, pour chaque solvant d'extraction mis en oeuvre, le coefficient de partage calculé entre la phase organique et la phase aqueuse, c'est-à-dire le rapport entre la concentration de chaque impureté dans la phase organique et sa concentration dans la phase aqueuse, après extraction.

Lorsque, après extraction, la concentration en une impureté dans la phase aqueuse est inférieure à la limite de détection analytique, une valeur minimale de coefficient de partage est indiquée, correspondant au rapport entre la concentration de cette impureté dans le solvant organique et sa limite de détection dans la phase aqueuse. Dans ce cas, la valeur est précédée du signe >.

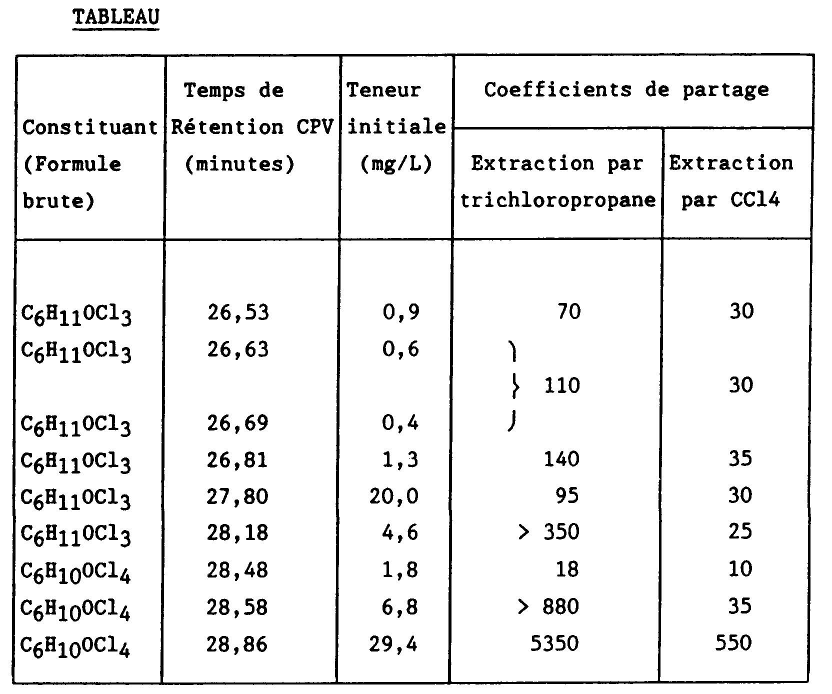

On observe que par rapport à une extraction par du tétrachlorométhane, l'extraction par du 1,2,3-trichloropropane permet de réduire la teneur en impuretés chloroaliphatiques d'un facteur 2 à 25.

## Revendications

1. Procédé de production d'épichlorhydrine au départ de chlorure d'allyle, comprenant les étapes suivantes :
a) hypochloration du chlorure d'allyle pour former une solution aqueuse brute de dichloropropanols qui contient en outre de faibles quantités d'impuretés organiques chlorées, notamment du 1,2,3-trichloropropane et des impuretés chloroaliphatiques.
b) extraction de cette solution aqueuse brute de dichloropropanols avec un solvant organique d'extraction recyclé, riche en 1,2,3-trichloropropane, en quantité suffisante pour obtenir une phase organique qui comprend principalement le 1,2,3-trichloropropane enrichi en impuretés chloroaliphatiques et une phase aqueuse contenant la majeure partie des dichloropropanols, fortement appauvrie en impuretés chloroaliphatiques.
c) déshydrochloration des dichloropropanols en épichlorhydrine par introduction d'un composé basique dans la phase aqueuse obtenue à l'étape b), entraînement à la vapeur de l'épichlorhydrine formée pour obtenir de l'épichlorhydrine brute et rejet des effluents aqueux.
d) séparation, par distillation fractionnée, de l'épichlorhydrine brute formée en c) en une fraction légère, en une fraction intermédiaire constituée d'épichlorhydrine purifiée et en une fraction lourde résiduelle et recyclage de la fraction lourde résiduelle de cette distillation à l'étape b) d'extraction où elle constitue au moins partiellement le solvant organique d'extraction recyclé riche en 1,2,3-trichloropropane.
e) lavage avec de l'eau, de la phase organique obtenue à l'étape b), pour obtenir, après séparation des phases, une eau de lavage contenant la majeure fraction des dichloropropanols présents dans ladite phase organique et un extrait organique lavé, contenant nettement moins de dichloropropanols qu'à l'alimentation, l'eau de lavage chargée en dichloropropanols étant recyclée à au moins une des étapes a) d'hypochloration, b) d'extraction ou c) de déshydrochloration.
f) séparation, par distillation fractionnée, dudit extrait organique lavé de l'étape e), en une coupe de tête envoyée à l'étape d) de séparation par distillation de l'épichlorhydrine brute, en une coupe moyenne constituée de 1,2,3-trichloropropane purifié et en une coupe de queue, constituée essentiellement par les impuretés chloroaliphatiques.

2. Procédé selon la revendication 1 dans lequel une partie du 1,2,3-trichloropropane purifié, correspondant à la coupe moyenne obtenue à l'étape f) de séparation par distillation de l'extrait organique lavé, est ajoutée à la fraction lourde résiduelle obtenue à l'étape d) de séparation par distillation de l'épichlorhydrine brute, le mélange obtenu étant recyclé à l'étape b) d'extraction.

3. Procédé selon la revendication 1 ou 2 dans lequel la séparation par distillation de l'extrait organique lavé, effectuée à l'étape f), est menée de manière telle que la coupe de tête envoyée à l'étape d) de séparation par distillation de l'épichlorhydrine contienne de 50 à 98 % de 1,2,3-trichloropropane.

4. Procédé selon une quelconque des revendications 1 à 3 dans lequel l'étape b) d'extraction est menée en continu avec un rapport entre le débit de la solution d'extraction riche en 1,2,3-trichloropropane et le débit de la solution aqueuse brute de dichloropropanols supérieur à 0,001 et inférieur à 0,1.

## Claims

1. Process for the production of epichlorohydrin starting from allyl chloride, comprising the following stages:
a) hypochlorination of allyl chloride to form a crude aqueous solution of dichloropropanols which additionally contains small amounts of chlorinated organic impurities, especially 1,2,3-trichloropropane and chloroaliphatic impurities.
b) extraction of this crude aqueous solution of dichloropropanols with a recycled organic extraction solvent, which is rich in 1,2,3-trichloropropane, in an amount sufficient to obtain an organic phase which mainly comprises 1,2,3-trichloropropane enriched in chloroaliphatic impurities and an aqueous phase containing the majority of the dichloropropanols, highly impoverised in chloroaliphatic impurities.
c) dehydrochlorination of the dichloropropanols to epichlorohydrin by introduction of a basic compound into the aqueous phase obtained in stage b), steam distillation of the epichlorohydrin formed to obtain crude epichlorohydrin and discarding of the aqueous effluents.
d) separation, by fractional distillation, of the crude epichlorohydrin formed in c) into a light fraction, into an intermediate fraction consisting of purified epichlorohydrin and into a residual heavy fraction, and recycling of the residual heavy fraction from this distillation to the extraction stage b) where it at least partially constitutes the recycled organic extraction solvent rich in 1,2,3-trichloropropane.
e) washing of the organic phase obtained in stage b) with water to obtain, after separation of the phases, a washing water containing the majority of the dichloropropanols present in the said organic phase and a washed organic extract, containing markedly less dichloropropanols than as supplied, the washing water laden with dichloropropanols being recycled to at least one of the a) hypochlorination, b) extraction or c) dehydrochlorination stages.
f) separation, by fractional distillation, of the said washed organic extract from stage e) into a head cut conveyed to the separation stage d) of the crude epichlorohydrin by distillation, into a middle cut consisting of purified 1,2,3-trichloropropane and into a bottom cut consisting essentially of the chloroaliphatic impurities.

2. Process according to Claim 1, in which part of the purified 1,2,3-trichloropropane, corresponding to the middle cut obtained in the separation stage f) by distillation of the washed organic extract, is added to the residual heavy fraction obtained in the separation stage d) of the crude epichlorohydrin by distillation, the mixture obtained being recycled to the extraction stage b).

3. Process according to Claim 1 or 2, in which the separation by distillation of the washed organic extract, carried out in stage f), is carried out such that the head cut conveyed to the separation stage d) of the epichlorohydrin by distillation contains from 50 to 98 % of 1,2,3-trichloropropane.

4. Process according to any one of Claims 1 to 3, in which the extraction stage b) is carried out continuously with a ratio between the flow rate of the extraction solution rich in 1,2,3-trichloropropane and the flow rate of the crude aqueous solution of dichloropropanols which is greater than 0.001 and less than 0.1.

## Patentansprüche

1. Verfahren zur Herstellung von Epichlorhydrin aus Allylchlorid, das die folgenden Schritte umfaßt:
a) Hypochlorierung des Allylchlorids zur Bildung einer rohen wäßrigen Lösung von Dichlorpropanolen, die außerdem geringe Mengen an chlorierten organischen Verunreinigungen, insbesondere 1,2,3-Trichlorpropan und chloraliphatische Verunreinigungen, enthält.
b) Extraktion dieser rohen wäßrigen Lösung von Dichlorpropanolen mit einem an 1,2,3-Trichlorpropan reichen rückgeführten organischen Extraktionslösungsmittel in ausreichender Menge, um eine organische Phase, die hauptsächlich das mit chloraliphatischen Verunreinigungen angereicherte 1,2,3-Trichlorpropan umfaßt, und eine an chloraliphatischen Verunreinigungen stark verarmte wäßrige Phase, die den Hauptteil der Dichlorpropanole enthält, zu erhalten.
c) Dehydrochlorierung der Dichlorpropanole zu Epichlorhydrin durch Zugabe einer basischen Verbindung in die in Schritt b) erhaltene wäßrige Phase, Wasserdampfdestillation des gebildeten Epichlorhydrins, um rohes Epichlorhydrin zu erhalten, und Verwerten der wäßrigen Abwässer.
d) Trennung des in c) gebildeten rohen Epichlorhydrins durch fraktionierte Destillation in eine leichtsiedende Fraktion, in eine aus gereinigtem Epichlorhydrin bestehende Zwischenfraktion, und in eine schwersiedende Restfraktion und Rückführung der schwersiedenden Restfraktion dieser Destillation in den Schritt b) der Extraktion, wo sie wenigstens teilweise das an 1,2,3-Trichlorpropan reiche rückgeführte organische Extraktionslösungsmittel bildet.
e) Waschen der in Schritt b) erhaltenen organischen Phase mit Wasser, um nach Trennung der Phasen ein Waschwasser, das die Hauptfraktion der in besagter organischer Phase vorhandenen Dichlorpropanole enthält, und einen gewaschenen organischen Extrakt, der deutlich weniger Dichlorpropanole als die Einspeisung enthält, zu erhalten, wobei das mit Dichlorpropanolen beladene Waschwasser in wenigstens einen der Schritte a) der Hypochlorierung, b) der Extraktion oder c) der Dehydrochlorierung rückgeführt wird.
f) Trennung des in Schritt e) gewaschenen besagten organischen Extrakts durch fraktionierte Destillation in eine Kopffraktion, die in den Schritt d) der Abtrennung des rohen Epichlorhydrins durch Destillation geschickt wird, in eine mittlere Fraktion, die aus gereinigtem 1,2,3-Trichlorpropan besteht, und in eine Schwanzfraktion, die im wesentlichen aus den chloraliphatischen Verunreinigungen besteht.

2. Verfahren gemäß Anspruch 1, bei dem ein Teil des gereinigten 1,2,3-Trichlorpropans, der der mittleren Fraktion, die im Schritt f) der Trennung des gewaschenen organischen Extrakts durch Destillation erhalten wird, entspricht, zu der schwersiedenden Restfraktion, die im Schritt d) der Abtrennung von rohem Epichlorhydrin durch Destillation erhalten wird, hinzugefügt wird, wobei das erhaltene Gemisch in den Schritt b) der Extraktion rückgeführt wird.

3. Verfahren gemäß Anspruch 1 oder 2, bei dem die Trennung des gewaschenen organischen Extrakts durch Destillation, die im Schritt f) ausgeführt wird, so geführt wird, daß die Kopffraktion, die in den Schritt d) der Abtrennung von Epichlorhydrin durch Destillation geschickt wird, 50 bis 98% 1,2,3-Trichlorpropan enthält.

4. Verfahren gemäß einem der Ansprüche 1 bis 3, bei dem der Schritt b) der Extraktion kontinuierlich mit einem Verhältnis zwischen dem Durchsatz der an 1,2,3-Trichlorpropan reichen Extraktionslösung und dem Durchsatz der rohen wäßrigen Lösung von Dichlorpropanolen, das größer als 0,001 und kleiner als 0,1 ist, geführt wird.
